# EUROPÄISCHE PATENTSCHRIFT

(11) **EP 2 809 410 B1**
(45) Veröffentlichungstag und Bekanntmachung des Hinweises auf die Patenterteilung: **20.01.2016**
(21) Anmeldenummer: 12703449.4
(22) Anmeldetag: 02.02.2012
(51) Int. Cl.: C07D 311/40, C07D 311/62, C08B 37/00, B01D 11/02

(54) **VERFAHREN ZUR EXTRAKTION VON HOLZ**
METHOD FOR AN EXTRACTION FROM WOOD
PROCÉDÉ D'EXTRACTION DU BOIS

(43) Veröffentlichungstag der Anmeldung: 10.12.2014
(73) Patentinhaber: LDA AG, 8832 Wollerau (CH)
(72) Erfinder: WIESBECK, Franz, CH-8835 Feusisberg (CH)
(74) Vertreter: Hoefer & Partner Patentanwälte mbB
(86) Internationale Anmeldenummer: PCT/EP2012/000476
(87) Internationale Veröffentlichungsnummer: WO 2013/113331

(56) Entgegenhaltungen:
- CA-A1- 1 032 826
- US-A- 1 762 785
- US-A- 3 944 677

## Beschreibung

Vorliegende Erfindung betrifft ein Verfahren zur Extraktion von Holz. Insbesondere wird Taxifolin und/oder Arabinogalactan aus dem Holz extrahiert.

Üblicherweise wird Holz vor dem Extrahieren gehackt oder geschreddert. Die dabei entstehenden Hackschnitzel bzw. das Schreddergut wird in einem Perkolator gefüllt und das entsprechende Extraktionsfluid eingeleitet.

Ein solches Verfahren wird im US 1762785 beschrieben.

Es ist Aufgabe vorliegender Erfindung, ein Verfahren zur Extraktion von Holz bereitzustellen, das bei kostengünstiger und prozesssicherer Durchführung eine möglichst schnelle, ertragreiche und energieeffiziente Extraktion der entsprechenden Stoffe aus dem Holz ermöglicht. Insbesondere soll dabei mit Ethanol möglichst effizient Taxifolin und/oder mit Wasser möglichst effizient Arabinogalactan aus dem Holz extrahiert werden.

Die Lösung der Aufgabe erfolgt durch die Merkmale des Anspruchs 1. Die abhängigen Ansprüche haben bevorzugte Weiterbildungen der Erfindung zum Gegenstand.

Somit wird die Aufgabe gelöst durch ein Verfahren zur Extraktion von Holz, umfassend die folgenden Schritte:
(i) Bereitstellen von Holzpartikeln, zumindest einer ersten Klasse und einer zweiten Klasse,
(ii) Aufschichten einer Feinschicht, bestehend aus den Holzpartikeln der ersten Klasse über einer weiteren Schicht, bestehend aus den Holzpartikeln der zweiten Klasse.
(iii) Extrahieren der Holzpartikel mit einem flüssigen Extraktionsmittel (auch: Extraktionsfluid oder Lösungsmittel), wobei das Extraktionsmittel von oben nach unten zunächst durch die Feinschicht und anschließend durch die weitere Schicht fließt. Die Holzpartikel der beiden Klassen unterscheiden sich erfindungsgemäß in ihrer Feinheit. Die Holzpartikel der ersten Klasse sind feiner als die Holzpartikel der zweiten Klasse. Somit weist die Feinschicht eine geringere Durchströmbarkeit als die weitere Schicht auf. Die "Durchströmbarkeit" einer Schicht beschreibt die Menge an Extraktionsmittel, die bei gleicher Schichtdicke, gleichem Druck und gleicher Zeit, durch die Schicht läuft. Zum Vergleich der Durchströmbarkeit unterschiedlicher Schichten, muss die Durchflussmenge an Extraktionsmittel durch Schichten mit gleicher Schichtdicke bei gleichem Druck und über die gleiche Zeit gemessen werden. Eine geringere Durchflussmenge bedeutet eine geringere Durchströmbarkeit der jeweiligen Schicht. Alternativ kann die "Durchströmbarkeit" unterschiedlicher Schichten auch bei konstanter Durchflussmenge und variablem Druck gemessen werden. Ist ein höherer Druck für dieselbe Durchflussmenge nötig, so spricht man von einer geringeren Durchströmbarkeit der Schicht. Bei der Durchführung des erfindungsgemäßen Verfahrens werden die einzelnen Schichten vorzugsweise als horizontale Schichten in einem Perkolator angeordnet. Das Extraktionsmittel wird dabei von oben in den Perkolator eingeleitet. Die Feinschicht ist dabei derart angeordnet, dass das Extraktionsfluid zwangsläufig zunächst durch die Feinschicht und anschließend durch die weitere Schicht mit den Holzpartikeln der zweiten Klasse fließt. Die geringere Durchströmbarkeit der Feinschicht erhöht somit den Druck über der Feinschicht und reduziert einen Druckabfall über die Höhe der Schichten. Dadurch verbessert sich die Zirkulierbarkeit des Extraktionsmittels.

Sehr kleine Holzpartikel, insbesondere unter 1 mm, sind aus verfahrenstechnischer Sicht wegen der schlechten Durchströmbarkeit nicht erwünscht, lassen sich aber bei der Zerkleinerung nicht vermeiden. Wesentlicher Vorteil der erfindungsgemäßen Feinschicht ist die Vermeidung eines zu großen Druckabfalls bei der Extraktion. Insbesondere wird mittels der Feinschicht ein Druckabfall über dem Perkolatorbett reduziert und damit die Zirkulierbarkeit des flüssigen Extraktionsmittels verbessert. Das flüssige Extraktionsmittel durchläuft den Perkolator insbesondere in mehreren Zyklen. Dabei durchläuft das Extraktionsmittel bei jedem Durchlauf die Feinschicht, so dass die feinen Holzpartikel und etwaige Verunreinigungen in der Feinschicht hängen bleiben. Infolge dessen ist das letztendlich aus dem Perkolator abgelassene Extraktionsmittel möglichst frei von feinen Holzpartikeln und Verunreinigungen. Bevorzugt muss das Extraktionsmittel nach Verlassen des Perkolators nicht mehr gefiltert werden bzw. reduziert sich aufgrund des erfindungsgemäßen Verfahrens die Standzeit bzw. die Dimension einer dem Perkolator nachgeschalteten Filtereinheit erheblich. Die Feinschicht dient somit auch als Vorfilter.

In bevorzugter Ausführung wird zwischen feinen und groben Holzpartikeln mittels des Medianwertes der Größenverteilung unterschieden. So ist bevorzugt vorgesehen, dass der Medianwert (erster Medianwert) der Partikelgrößenverteilung aller Holzpartikel in der ersten Klasse niedriger ist als der Medienwert (zweiter Medianwert) der Partikelgrößenverteilung aller Holzpartikel in der zweiten Klasse.

Die Partikelgröße ist insbesondere die nominelle Siebgröße des Holzpartikels. Die nominelle Siebgröße des Holzpartikels ist die Lochgröße eines Siebes, durch das noch zumindest ein Massenanteil von 95% des Materials hindurchgeht.

Besonders bevorzugt wird zur Bestimmung der nominellen Siebgröße ein Siebverfahren gemäß der DIN EN 15149-1: 2010 bzw. DIN EN 15149-2: 2010 verwendet. Gemäß diesen Normen ist zur Bestimmung der nominellen Siebgröße und der entsprechenden Größenverteilung eine geeignete Anzahl übereinander gestapelter Siebe zu verwenden. Dabei nimmt der Lochdurchmesser bzw. die Maschenweite der einzelnen Siebe von oben nach unten ab. Es erfolgt eine horizontale ein- oder zweidimensionale Rüttelung des Siebpaketes. Ferner definieren diese beiden Normen auch den Medianwert der Partikelgrößenverteilung. Demgemäß ist der Medienwert derjenige Wert, der eine Verteilung in zwei gleich große Teile aufteilt und der graphisch den Schnittpunkt der kumulativen Größenverteilungskurve mit der 50%-Horizontalen darstellt. Bevorzugt werden die Holzpartikel mit einem kleineren Medianwert der Partikelgrößenverteilung als "feinere Holzpartikel" angesehen und somit für die Feinschicht verwendet.

Besonders bevorzugt ist der Medianwert (erster Medianwert) in der ersten Klasse kleiner oder gleich 3mm, vorzugsweise kleiner oder gleich 2mm, besonders vorzugsweise kleiner oder gleich 1 mm, weiter besonders vorzugsweise kleiner oder gleich 0.5mm. Je kleiner der Medianwert in der ersten Klasse ist, umso langsamer durchströmt das Lösungsmittel die Feinschicht. Somit kann durch die Auswahl des Medianwertes und der Höhe der Feinschicht der Druckabfall eingestellt werden.

Das Verfahren umfasst des Weiteren bevorzugt folgende Schritte: Zum Bereitstellen der Holzpartikel wird zunächst Holz in Holzpartikel verschiedener Größe zerkleinert. Das Zerkleinern erfolgt durch Hacken und/oder Schreddern und/oder Mahlen. Daraufhin wird das geschredderte oder zerhackte oder gemahlene Holz klassiert. Das Klassieren erfolgt insbesondere durch Sieben. Bei dem Klassieren wird zumindest zwischen Holzpartikeln der ersten Klasse und Holzpartikeln der zweiten Klasse unterschieden. Ausschlaggebend bei dem Klassieren ist, dass ausreichend feine Holzpartikel der ersten Klasse abgeschieden werden. Dabei ist es nicht notwendig, dass sämtliche feinen Holzpartikel von der zweiten Klasse abgeschieden werden.

Zusätzlich zu dem Klassieren des Hack- oder Schredder- oder Mahlgutes können anderweitig hergestellte Sägespäne und/oder Hobelspäne der ersten Klasse zugeführt werden. Des Weiteren ist es alternativ zum Klassieren auch möglich, dass die Feinschicht lediglich aus anderweitig hergestellten Sägespänen und/oder Hobelspänen und/oder Holzmehl gebildet wird.

In besonders bevorzugter Ausführung ist vorgesehen, dass zwischen der ersten Klasse und der zweiten Klasse mit einem Sieb mit einer bestimmten Maschenweite oder einem bestimmten Lochdurchmesser klassiert wird. Die Maschenweite oder der Lochdurchmesser liegen dabei bevorzugt zwischen 0,5 mm und 7 mm. Der Versuch hat gezeigt, dass sich eine praktikable Trennung zwischen den beiden Klassen insbesondere mit einer Maschenweite bzw. einem Lochdurchmesser zwischen 1 mm und 6 mm, vorzugsweise zwischen 1 mm und 4 mm, realisieren lässt. Je nach Wasser- und Harzgehalt und Art der Zerkleinerung des Holzes setzt sich das unklassierte Material bezüglich der Partikelgössenverteilungen unterschiedlich zusammen. Dementsprechend wird hier mit den bevorzugten Maschenweiten oder Lochdurchmessern ein Absieben der ersten Klasse erreicht.

Ferner kann zwischen feinen und groben Holzpartikeln anhand der Menge übergroßer Holzpartikel in den Klassen unterschieden werden. Es ist von Vorteil, wenn in der Feinschicht nicht zu viele übergroße Holzpartikel vorhanden sind. Dadurch wird gewährleistet, dass über die gesamte Feinschicht die Durchströmbarkeit konstant ist. Deshalb ist es bevorzugt vorgesehen, dass über 70% Massenanteil der Holzpartikel in der ersten Klasse höchstens eine nominelle Siebgröße von 4 mm, vorzugsweise 2mm, besonders vorzugsweise 1 mm, aufweisen. Beim Zerkleinern des Holzes hängt die Menge der Holzpartikel der ersten Klasse z.B. vom Wasser- und Harzgehalt sowie von der verwendeten Zerkleinerungstechnik ab. Somit kann die Größenverteilung der Holzpartikel beim Zerkleinern nicht beliebig eingestellt werden.

Vorteilhaft läuft das Extraktionsmittel relativ definiert und ungehindert durch die weitere Schicht. Deshalb soll die zweite Klasse möglichst wenig feine Holzpartikel aufweisen. Es ist bevorzugt vorgesehen, dass über 30% Massenanteil der Holzpartikel in der zweiten Klasse mindestens eine nominelle Siebgröße von 2 mm, vorzugsweise 3 mm, aufweisen.

Ferner ist bevorzugt vorgesehen, dass der Feinheitsgrad der Holzpartikel in der Feinschicht ausreichend gering ist und möglichst wenig große und übergroße Holzpartikel vorhanden sind. Deshalb wird für die nominelle Siebgröße der Holzpartikel von über 60%, vorzugsweise über 70%, besonders vorzugsweise über 80%, Massenanteil aller Holzpartikel, welche in der ersten Klasse sind, eine Obergrenze definiert. In der weiteren Schicht sollte eine ausreichende Menge an großen Holzpartikeln vorhanden sein, um hier einen definierten und ausreichenden Durchfluss des Extraktionsmittels zu gewährleisten. Für die nominelle Siebgröße der Holzpartikel von über 10%, vorzugsweise über 20%, besonders vorzugsweise über 30%, Massenanteil aller Holzpartikel der zweiten Klasse, wird eine Untergrenze definiert.

Bevorzugt ist vorgesehen, dass die Obergrenze der ersten Klasse gleich oder niedriger der Untergrenze der zweiten Klasse ist. Besonders bevorzugt liegt die Obergrenze der ersten Klasse bei 4 mm, vorzugsweise bei 2 mm, besonders vorzugsweise bei 1 mm.

Je nach Beschaffenheit des zerhackten, geschredderten oder gemahlenen Holzes, entsteht mehr oder weniger Material der ersten Klasse. Bevorzugt wird eine Schichthöhe der Feinschicht vorgegeben. Zum einen soll die Feinschicht nicht zu hoch sein, um einen ausreichenden Durchfluss des Extraktionsmittels zu gewährleisten. Auf der anderen Seite ist eine gewisse Schichthöhe der Feinschicht notwendig, um einen ausreichenden Filtereffekt zu erzielen und um einen zu großen Druckabfall zu vermeiden. Als vorteilhaft hat sich eine Schichthöhe der Feinschicht von 5% bis 70%, vorzugsweise 5% bis 50%, besonders vorzugsweise 5% bis 40%, bezogen auf die Gesamthöhe aller Schichten, herausgestellt.

Bevorzugt erfolgt das Aufschichten der Holzpartikel in einem Perkolator. Das erfindungsgemäße Verfahren kommt bevorzugt bei der Extraktion von sehr großen Mengen zum Einsatz. Die Schichthöhe der Feinschicht liegt hierbei vorteilhafterweise bei 0,5 m, vorzugsweise bei 1 bis 3 m.

Bevorzugt erfolgt eine Extraktion von Lärchenholz. Besonders bevorzugt werden lediglich Stammkernholz und/oder der Wurzelstock der Lärche verwendet.

Des Weiteren erfolgt mit dem erfindungsgemäßen Verfahren insbesondere eine Extraktion von Taxifolin aus dem Holz, mit einem organischen Lösungsmittel, vorzugsweise mit Ethanol. Zusätzlich oder alternativ erfolgt eine Extraktion von Arabinogalactan aus dem Holz, vorzugsweise mit Wasser als Lösungsmittel.

Die Probennahme aus dem geschredderten oder gehackten oder gemahlenen Holz erfolgt bevorzugt nach DIN EN 14778: 2011 und die Bestimmung der Größe der Holzpartikel sowie die Partikelgrößenverteilung nach DIN EN 15149-1: 2010 bzw. DIN EN 15149-2: 2010.

Ferner können auch mehrere Feinschichten oder mehrere weitere Schichten beliebig gestapelt werden.

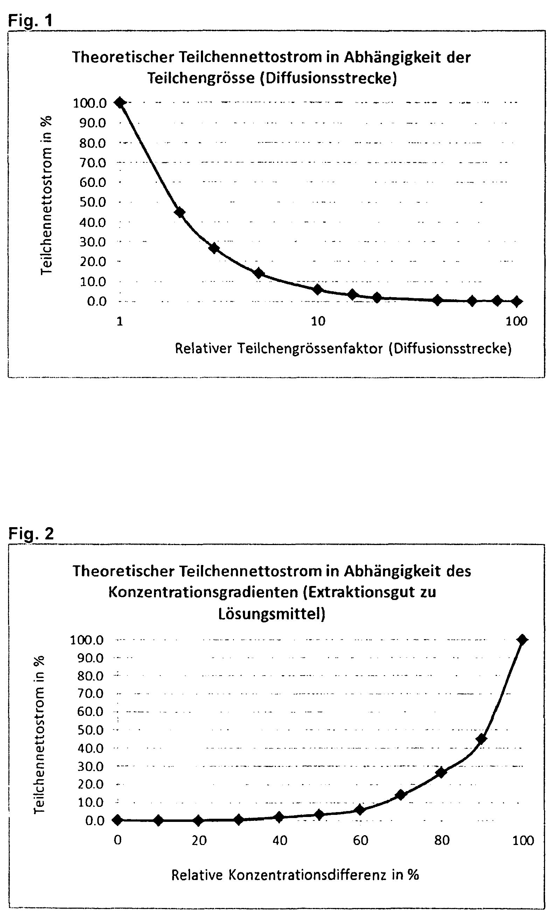

Die Extraktion basiert theoretisch auf den Fick'schen Diffusionsgesetzten. Hieraus kann zum einen abgeleitet werden, dass je kürzer die erforderliche Diffusionsstrecke aus dem Extraktionsgut in das Lösungsmittel ist, desto rascher kann das gewünschte Produkt aus dem Extraktionsgut extrahiert werden. Hieraus lässt sich folgern, dass je kleiner die Partikel des verwendeten Extraktionsgutes sind, umso geringer ist die zu überwindende Diffusionsstrecke und damit umso grösser der theoretische Teilchennettostrom in das Lösungsmittel. Fig.1 zeigt hierzu einen Verlauf des theoretischen Teilchennettostroms in Abhängigkeit der Teilchengröße.

Zum anderen kann abgeleitet werden, dass je grösser das erreichte Konzentrationsgefälle der Produktkonzentration im Partikel zum Lösungsmittel ist, desto rascher kann ebenfalls das gewünschte Produkt aus dem Extraktionsgut extrahiert werden.

Hieraus lässt sich folgern, dass je höher die Zirkulationsrate oder die verwendete Menge des verwendeten Lösungsmittels ist, umso höher ist das die Diffusion antreibende Konzentrationsgefälle und damit umso größer der theoretische Teilchennettostrom in das Lösungsmittel. Fig. 2 zeigt hierzu einen Verlauf des theoretischen Teilchennettostroms in Abhängigkeit des Konzentrationsgradienten.

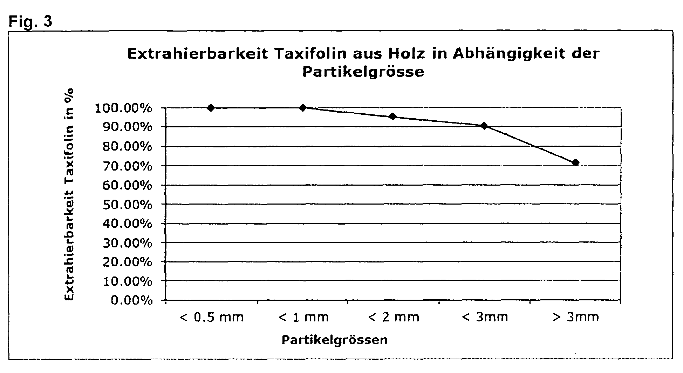

Es wurde eine Versuchreihe zur Extrahierbarkeit des Taxifolins aus Lärchenholz in Abhängigkeit der verwendeten Holzpartikelgrößen durchgeführt. Die Extraktionszeiten, Extraktionstemperatur, Durchmischung, sowie das Mengenverhältnis an eingesetztem Lösungsmittel zu Extraktionsgut wurde hierbei konstant gehalten. Fig. 3 zeigt den Verlauf der Extrahierbarkeit von Taxifolin aus Lärchenholz in Abhängigkeit der nominellen Siebgröße der Holzpartikel.

## Patentansprüche

1. Verfahren zur Extraktion von Holz, umfassend die folgenden Schritte:
- Bereitstellen von Holzpartikeln zumindest einer ersten Klasse und einer zweiten Klasse,
- Aufschichten einer Feinschicht bestehend aus den Holzpartikeln der ersten Klasse über einer weiteren Schicht bestehend aus den Holzpartikeln der zweiten Klasse, und
- Extrahieren der Holzpartikel mit einem flüssigen Extraktionsmittel, wobei das Extraktionsmittel von oben nach unten zunächst durch die Feinschicht und anschließend durch die weitere Schicht fließt,
- wobei die Holzpartikel der ersten Klasse feiner sind als die Holzpartikel der zweiten Klasse und somit die Feinschicht eine geringere Durchströmbarkeit aufweist als die weitere Schicht.

2. Verfahren nach Anspruch 1, **dadurch gekennzeichnet, dass** ein erster Medianwert der Partikelgrößenverteilung aller Holzpartikel in der ersten Klasse niedriger ist als ein zweiter Medianwert der Partikelgrößenverteilung aller Holzpartikel in der zweiten Klasse, wobei insbesondere die Partikelgröße die nominelle Siebgröße ist.

3. Verfahren nach Anspruch 2, **dadurch gekennzeichnet, dass** der erste Medianwert kleiner oder gleich 3mm, vorzugsweise kleiner oder gleich 2mm, besonders vorzugsweise kleiner oder gleich 1 mm, ist.

4. Verfahren nach einem der vorhergehenden Ansprüche, **dadurch gekennzeichnet, dass** das Bereitstellen von Holzpartikeln folgende Schritte umfasst:
- Zerkleinern von Holz in Holzpartikel verschiedener Größe, und
- Klassieren, insbesondere Sieben, der Holzpartikel in zumindest die erste Klasse und die zweite Klasse.

5. Verfahren nach Anspruch 4, **dadurch gekennzeichnet, dass** zwischen der ersten Klasse und der zweiten Klasse mit einem Sieb mit einer Maschenweite oder einem Lochdurchmesser zwischen 0,5mm und 7mm, vorzugsweise zwischen 1 mm und 6mm, besonders vorzugsweise zwischen 1 mm und 4mm, klassiert wird.

6. Verfahren nach einem der vorhergehenden Ansprüche, **dadurch gekennzeichnet, dass** über 70% Massenanteil der Holzpartikel in der ersten Klasse eine nominelle Siebgröße von höchstens 4mm, vorzugsweise höchstens 2mm, besonders vorzugsweise höchstens 1mm, aufweisen.

7. Verfahren nach einem der vorhergehenden Ansprüche, **dadurch gekennzeichnet, dass** über 30% Massenanteil der Holzpartikel in der zweiten Klasse ein nominelle Siebgröße von mindestens 2mm, vorzugsweise mindestens 3mm, aufweisen.

8. Verfahren nach einem der vorhergehenden Ansprüche, **dadurch gekennzeichnet, dass** die nominelle Siebgröße der Holzpartikel von über 60%, vorzugsweise über 70%, besonders vorzugsweise über 80%, Massenanteil aller Holzpartikel in der ersten Klasse kleiner oder gleich einem ersten Grenzwert ist, und
dass die nominelle Siebgröße der Holzpartikel von über 10%, vorzugsweise über 20%, besonders vorzugsweise über 30%, Massenanteil aller Holzpartikel der zweiten Klasse größer oder gleich einem zweiten Grenzwert ist, wobei der erste Grenzwert gleich oder kleiner dem zweiten Grenzwert ist.

9. Verfahren nach Anspruch 8, **dadurch gekennzeichnet, dass** der erste Grenzwert bei einer nominellen Siebgröße von 4mm, vorzugsweise bei 2mm, besonders vorzugsweise bei 1 mm, liegt.

10. Verfahren nach einem der vorhergehenden Ansprüche, **dadurch gekennzeichnet, dass** eine Schichthöhe der Feinschicht 5% bis 70%, insbesondere 5% bis 50%, insbesondere 5% bis 40%, einer Gesamthöhe aller Schichten beträgt.

11. Verfahren nach einem der vorhergehenden Ansprüche, **dadurch gekennzeichnet, dass** die Holzpartikel in einem Perkolator aufgeschichtet werden, wobei die Schichthöhe der Feinschicht zumindest 0,5 m, vorzugsweise zumindest 1 m, beträgt.

12. Verfahren nach einem der vorhergehenden Ansprüche, **dadurch gekennzeichnet, dass** aus dem Holz Taxifolin, insbesondere mit Ethanol als Extraktionsmittel, und/oder Arabinogalactan, insbesondere mit Wasser als Extraktionsmittel, Extrahiert wird.

## Claims

1. Method for extracting wood, comprising the following steps:
- providing wood particles of at least a first class and a second class,
- layering a fine layer of the first-class wood particles over a further layer of the second-class wood particles, and
- extracting the wood particular using a liquid extraction agent, the extraction agent initially flowing through the fine layer from top to bottom and subsequently flowing through the further layer,
- the first-class wood particles being finer than the second-class wood particles, and the fine layer thus having a lower permeability than the further layer.

2. Method according to claim 1, **characterised in that** first median value of the particle size distribution of all of the first-class wood particles is lower than a second median value of the particle size distribution of all of the second-class wood particles, the particle size in particular being the nominal sieve mesh size.

3. Method according to claim 2, **characterised in that** the first median value is less than or equal to 3 mm, preferably less than or equal to 2 mm, particularly preferably less than or equal to 1 mm.

4. Method according to any of the preceding claims, **characterised in that** the preparation of wood particles comprises the following steps:
- comminuting wood into wood particles of various sizes, and
- classifying, in particularly sieving, the wood particles into at least the first class and the second class.

5. Method according to claim 4, **characterised in that** the classification between the first class and the second class takes place using a sieve having a mesh width or hole diameter of between 0.55 mm and 7 mm, preferably between 1 m and 6 mm, particularly preferably between 1 mm and 4 mm.

6. Method according to any of the preceding claims, **characterised in that** over 70 % by mass of the first-class wood particles have a nominal sieve mesh size of at most 4 mm, preferably at most 2 mm, particularly preferably at most 1 mm.

7. Method according to any of the preceding claims, **characterised in that** over 30 % by mass of the second-class wood particles have a nominal sieve mesh size of at least 2 mm, preferably at least 3 mm.

8. Method according to any of the preceding claims, **characterised in that** the nominal sieve mesh size of the wood particles of over 60 %, preferably over 70 %, particularly preferably over 80 % by mass of all of the first-class wood particles is less than or equal to a first limit, and **in that** the nominal sieve mesh size of the wood particles of over 10 %, preferably over 20 %, particularly preferably over 30 % by mass of all of the second-class wood particles is greater than or equal to a second limit, the first limit being less than or equal to the second limit.

9. Method according to claim 8, **characterised in that** the first limit is at a nominal mesh size of 4 mm, preferably at 2 mm, particular preferably at 1 mm.

10. Method according to any of the preceding claims, **characterised in that** the level height of the fine layer is 5 % to 70 %, in particular 5 % to 50 %, in particular 5 % to 40 %, of the total height of all of the layers.

11. Method according to any of the preceding claims, **characterised in that** the wood particles are layered in a percolator, the layer height of the fine layer being at least 0.5 m, preferably at least 1 m.

12. Method according to any of the preceding claims, **characterised in that** taxifolin, in particular using ethanol as an extraction agent, and/or arabinogalactan, in particular using water as an extraction agent, is extracted from the wood.

## Revendications

1. Procédé d'extraction à partir de bois, comprenant les étapes suivantes :
- mise à disposition de particules de bois au moins d'une première classe et d'une deuxième classe,
- empilement d'une couche fine constituée des particules de bois de la première classe sur une autre couche constituée des particules de bois de la deuxième classe, et
- extraction des particules de bois avec un produit d'extraction liquide, le produit d'extraction s'écoulant du haut vers le bas d'abord par la couche fine, puis par l'autre couche,
- les particules de bois de la première classe étant plus fines que les particules de bois de la deuxième classe et, par conséquent, la couche fine présentant une perméabilité plus faible que l'autre couche.

2. Procédé selon la revendication 1, **caractérisé en ce qu'**une première valeur médiane de la répartition granulométrique de toutes les particules de bois dans la première classe est plus faible qu'une deuxième valeur médiane de la répartition granulométrique de toutes les particules de bois dans la deuxième classe, en particulier la taille de particules étant la taille de tamis nominale.

3. Procédé selon la revendication 2, **caractérisé en ce que** la première valeur médiane est inférieure ou égale à 3 mm, de préférence inférieure ou égale à 2 mm, de manière particulièrement préférée inférieure ou égale à 1 mm.

4. Procédé selon l'une quelconque des revendications précédentes, **caractérisé en ce que** la mise à disposition de particules de bois comprend les étapes suivantes :
- broyage de bois en particules de bois de différentes tailles, et
- classement, en particulier tamisage, des particules de bois en au moins la première classe et la deuxième classe.

5. Procédé selon la revendication 4, **caractérisé en ce qu'**entre la première classe et la deuxième classe on classe avec un tamis avec une largeur de mailles ou un diamètre de trou entre 0,5 mm et 7 mm, de préférence entre 1 mm et 6 mm, de manière particulièrement préférée entre 1 mm et 4 mm.

6. Procédé selon l'une quelconque des revendications précédentes, **caractérisé en ce que** plus de 70 % du pourcentage en masse des particules de bois dans la première classe présentent une taille de tamis nominale de maximum 4 mm, de préférence de maximum 2 mm, de manière particulièrement préférée de maximum 1 mm.

7. Procédé selon l'une quelconque des revendications précédentes, **caractérisé en ce que** plus de 30 % du pourcentage en masse des particules de bois dans la deuxième classe présentent une taille de tamis nominale d'au moins 2 mm, de préférence d'au moins 3 mm.

8. Procédé selon l'une quelconque des revendications précédentes, **caractérisé en ce que** la taille de tamis nominale des particules de bois de plus de 60 %, de préférence de plus de 70 %, de manière particulièrement préférée de plus de 80 %, du pourcentage en masse de toutes les particules de bois dans la première classe est inférieure ou égale à une première valeur limite, et **en ce que** la taille de tamis nominale des particules de bois de plus de 10 %, de préférence de plus de 20 %, de manière particulièrement préférée de plus de 30 %, du pourcentage en masse de toutes les particules de bois de la deuxième classe est supérieure ou égale à une deuxième valeur limite, la première valeur limite étant inférieure ou égale à la deuxième valeur limite.

9. Procédé selon la revendication 8, **caractérisé en ce que** la première valeur limite se situe à une taille de tamis nominale de 4 mm, de préférence de 2 mm, de manière particulièrement préférée de 1 mm.

10. Procédé selon l'une quelconque des revendications précédentes, **caractérisé en ce qu'**une hauteur de couche de la couche fine est de 5 % à 70 %, en particulier de 5 % à 50 %, en particulier de 5 % à 40 %, d'une hauteur totale de toutes les couches.

11. Procédé selon l'une quelconque des revendications précédentes, **caractérisé en ce que** les particules de bois sont empilées dans un percolateur, la hauteur de couche de la couche fine étant d'au moins 0,5 m, de préférence d'au moins 1 m.

12. Procédé selon l'une quelconque des revendications précédentes, **caractérisé en ce qu'**on extrait à partir du bois de la taxifoline, en particulier avec de l'éthanol en tant que produit d'extraction, et/ou de l'arabinogalactane, en particulier avec de l'eau en tant que produit d'extraction.
